(19) Europäisches Patentamt
European Patent Office
Office européen des brevets

(11) Publication number: **0 371 823 A1**

(12) **EUROPEAN PATENT APPLICATION**

(21) Application number: **89312528.6**

(51) Int. Cl.[5]: **C07C 213/02, C07C 219/08**

(22) Date of filing: **30.11.89**

(30) Priority: **30.11.88 US 278297**

(43) Date of publication of application:
**06.06.90 Bulletin 90/23**

(84) Designated Contracting States:
**AT BE CH DE ES FR GB GR IT LI LU NL SE**

(71) Applicant: **POLYPURE INC.**
**2859 Paces Ferry Road**
**Atlanta Georgia 30339(US)**

(72) Inventor: **Chiang, William Gong-Jean**
**111 Woodberry Lane**
**Fayetteville, NY 13066(US)**
Inventor: **Jobbins, Richard McIntyre**
**1937 Annaste Lane**
**Marcellus, NY 13108(US)**

(74) Representative: **Brock, Peter William et al**
**URQUHART-DYKES & LORD 91 Wimpole Street**
**London W1M 8AH(GB)**

(54) **Crystalline 3-methacryloyloxy-2-hydroxypropyl-trimethylammonium chloride monomer.**

(57) The present invention provides a mixture of 3-methacryloyloxy-2-hydroxypropyltrimethylammonium chloride monomer and its isomer having a particle crystalline size of greater than about 40 microns. The crystalline monomers are directly prepared without an intervening recrystallization step. The monomers are useful in the preparation of water soluble polymers having antistatic, flocculation and water treatment properties.

EP 0 371 823 A1

# CRYSTALLINE 3-METHACRYLOYLOXY-2-HYDROXYPROPYLTRIMETHYLAMMONIUM CHLORIDE MONOMER

## BACKGROUND OF THE INVENTION

### Field of the Invention

The present invention relates to 3-methacryloyloxy-2-hydroxproppyltrimethylammonium chloride monomer and its isomer, 2-methacryloyloxy-3-hydroxypropyltriimethylammonium chloride monomer. These chlorides are collectively known as MAHTAC. The present invention also relates to a process for the preparation of the monomers.

### Description of the Prior Art

Since late 1970 the demand for cationic monomer used in cationic polymers in the water treatment chemical market has increased significantly. Commercially available monomers include 2-methacryloyloxyethyltrimethylammonium chloride (known in the art as Alcolac Sipomer® Q-6), and 2-acryloyloxyethyltrimethylammonium chloride (known in the art as Alcolac Sipomer® Q-9). Another suggested monomer is MAHTAC but these monomers are currently unavailable in commercial quantities.

## Summary of the Invention

The present invention provides a mixture of 3-methacryloyloxy-2-hydroxypropyltriimethylammonium chloride monomer and its isomer, 2-methacryloyloxy-3-hydroxypropyltrimethylammonium chloride monomer, wherein said crystalline monomers have an average particle size greater than 40 microns and a purity greater than 94%, said crystals being directly prepared without an intervening recrystallization step. These monomers are particularly useful in the preparation of water soluble polymers having antistatic, flocculation and water treatment properties.

The present invention also provides a process for the preparation of MAHTAC. The process comprises reacting 3-chloro-2-hydroxypropyl methacrylate and its isomer, 1-chloro-3-hydroxypropyl-2-methacrylate, with trimethylamine in the presence of MAHTAC.

In another embodiment, the process comprises the following steps. In step (a), methacrylic acid and epichlorohydrin are reacted in the presence of a catalytic amount of MAHTAC at a temperature and for a time sufficient to form a reaction product. In step (b), the reaction product of step (a) is then reacted with trimethylamine in the presence of a small amount of MAHTAC at a temperature and for a time sufficient to form MAHTAC. The reaction occurs in a non-solvent for the MAHTAC.

The chemical equations of the present process are expressed as follows. For step (a), the reaction is carried out in the presence of a small amount of MAHTAC and is represented by:

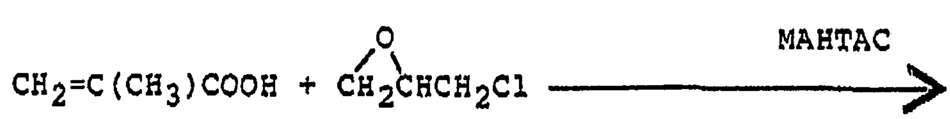


$CH_2{=}C(CH_3)COOCH_2C(OH)HCH_2Cl$ (CHPM)

+ $CH_2{=}C(CH_3)COOCH(CH_2OH\ CH_2Cl$ (CHPM isomer)

For step (b), carried out in a non-solvent for MAHTAC, the reaction equation is:

$$MAHTAC + CHPM/CHPM\ isomer + (CH_3)_3N \xrightarrow{non\text{-}solvent}$$

$$CH_2{=}C(CH_3)COOCH_2C(OH)HCH_2N^+(CH_3)_3Cl^-$$

$$+\ CH_2{=}C(CH_3)COOCH(CH_2OH)\ CH_2N^+(CH_3)_3Cl^-$$

As such, the present invention fills the need in the art for a 3-methacryloyloxy-2-hydroxypropyltrimethylammonium chloride monomer having a larger particle size and an improved process for the preparation thereof wherein the yield is higher than in current processes and the process is less expensive due to catalyst recycle.

Other objects and advantages of the present invention will become apparent from the following description, attached figures, and the appended claims.

## Brief Description of the Drawings

FIG. 1 is an optical micrograph of the 3-methacryloyloxy-2-hydroxypropyltrimethylammonium chloride monomer and 2-methacryloyloxy-3-hydroxypropyltrimethylammonium chloride monomer produced in Example 5 below.

FIG. 2 is an optical micrograph of the 3-methacryloyloxy-2-hydroxypropyltrimethylammonium chloride monomer and 2-methacryloyloxy-3-hydroxypropyltrimethylammonium chloride monomer produced in Example 6 below.

## Detailed Description of the Preferred Embodiments

To prepare the 3-methacryloyloxy-2-hydroxypropyltrimethylammonium chloride monomer, methacrylic acid (MAA) and epichlorohydrin (EPI) are reacted in the presence of a mixture of 3-methacryloyloxy-2-hydroxypropyltrimethylammonium chloride and its isomer as catalyst in step (a). Commercially available methacrylic acid and epichlorohydrin may be used in the present invention. Preferably, the molar ratio of methacrylic acid used to epichlorohydrin is about 0.5:1.0 to 1.0:0.5. If the ratio of epichlorohydrin to methacrylic acid used exceeds 1.0:0.5, polyepichlorohydrin forms. More preferably, the molar ratio of methacrylic acid to epichlorohydrin is about 0.8:1.0 to about 1.0:0.8. Most preferably, the molar ratio of methacrylic acid to epichlorohydrin is about 0.9:1.0 to about 1.0:1.0. The use of a slight excess epichlorohydrin is most preferred.

It has been discovered that a mixture of 3-methacryloyloxy-2-hydroxypropyltrimethylammonium chloride monomer and its isomer is a catalyst with advantages over triethylamine (a catalyst used in the art) in the formation of the chlorohydrin esters in step (a) of the monomer preparation.

Preferably, the molar ratio of the mixture of 3-methacryloyloxy-2-hydroxypropyltrimethylammonium chloride monomer and its isomer to methacrylic acid is about 0.01:1.0 to about 0.2:1.0. More preferably, the molar ratio of the MAHTAC mixture to methacrylic acid is about 0.01:1.0 to about 0.1:1.0. Most preferably, the molar ratio of the MAHTAC mixture to methacrylic acid is about 0.02:1.0 to about 0.04:1.0.

The methacrylic acid and epichlorohydrin are reacted in the presence of the MAHTAC mixture of chloride monomer and its isomer at a temperature and for a time sufficient to form a reaction product. Preferably, the methacrylic acid and epichlorohydrin are reacted in the presence of the MAHTAC mixture at a temperature of about 25 to about 100° C. More preferably, the temperature is about 50 to about 100° C; most preferably, the temperature is about 65 to about 90° C. Preferably, the reaction time is about 2 to about 48 hours. More preferably, the reaction time is about 3 to about 24 hours; most preferably, the reaction time is about 5 to about 7 hours. Reaction pressure should be about atmospheric.

Preferably, the reaction occurs in the presence of a polymerization inhibitor. Preferred polymerization inhibitors are p-methoxyphenol, hydroquinone and phenothiazine; these materials are available in commercial quantities. The polymerization inhibitors may be used in conventional amounts.

Although not wishing to be bound by theory, it is believed that the reaction mechanism of the step (a)

synthesis involves formation of an intermediate ionic complex [catalyst-acid] and then insertion of the epichlorohydrin epoxy ring into the complex [ionic complex-epichlorohydrin] via trans-opening of the epoxide ring.

Purification of the CHPM solution before the quaternization of step (b) is unnecessary. It has been found that although the use of a purified CHPM solution produces a high purity 3-methacryloyloxy-2-hydroxypropyltrimethylammonium chloride monomer, the overall process yield is lower and the reaction time is much longer than when an unpurified CHPM solution is used in step (b). If desired, crude CHPM solution can be purified to remove any residual methacrylic acid, epichlorohydrin and other impurities. To do this, the crude CHPM solution is first extracted with a saturated aqueous $NaHCO_3$ until the methacrylic acid is removed followed by vacuum distillation to remove any residual epichlorohydrin and other residual impurities.

For step (b), the reaction product of step (a) is reacted with trimethylamine in the presence of the MAHTAC at a temperature and for a time sufficient to form 3-methacryloyloxy-2-hydroxypropyltrimethylammonium chloride and its isomer 2-methacryloyloxy-3-hydroxypropyl trimethylammonium chloride monomer. Preferably, the molar ratio of the reaction product to the trimethylamine is about 1.0:1.0 to about 1.0:2.0. More preferably, the molar ratio of the reaction product to the trimethylamine is about 1.0:1.0 to about 1.0:1.2.

It has been unexpectedly discovered that the presence of additional amounts of MAHTAC acts as a nucleus or seed in the reaction mixture of step (b) to influence the particle size distribution of the product monomer. In step (b) the mixture of MAHTAC also acts as a catalyst. Although not wishing to be bound by theory, it is believed that the catalytic action of the monomer loosens the C-C1 bond of CHPM by interaction between its positive nitrogen and the negative chlorine atom of CHPM. Preferably, the molar ratio of the reaction product to the MAHTAC mixture is about 1.0:0.01 to about 1.0:0.50. More preferably, the molar ratio of the reaction product to the MAHTAC mixture is about 1.0:0.03 to about 1.0:0.15. Although not wishing to be bound by theory, it is believed that the reaction mechanism of the quaternization step follows nucleophilic substitution including $S_N2$ displacement and intramolecular displacement of the chloride ion.

The nature of the reaction medium affects the reaction rate and yield of the MAHTAC reaction. Since the reaction between two neutral species leads to ionic products, the rate is enhanced by ionizing media like polar solvent. Unfortunately, polar protic media like water promote side reactions such as hydrolysis and transesterification, particularly in the basic environment created by the presence of trimethylamine. Media such as low molecular weight alcohols become involved in transesterification reactions with the reactants and products and are also not useful. Poorly ionizing or nonionizing media like ethers, and hydrocarbons result in very slow reactions. A useful solvent would be polar but aprotic - a solvent for the CHPM but a poor solvent for the MAHTAC (about 3% at room temperature). Acetonitrile is one example of such a non-solvent.

In the present $S_N2$ reaction, the reaction between a neutral substrate (CHPM) and a neutral nucleophile (TMA) produces ions and is therefore accelerated by increasing the ionizing power of the reaction medium. Preferred non-solvents include tetrahydrofuran; acetone; acetonitrile; propionitrile; butyronitrile; dimethyl formamide; dimethyl sulfoxide; methyl ethyl ketone; ethylacetate; and 1,1,2-trichloroethene. The more preferred non-solvents are acetonitrile; acetone; methyl ethyl ketone; tetrahydrofuran; ethylacetate; and 1,1,2-trichloroethene. The most preferred non-solvent is acetonitrile. The non-solvent is used in amount of about 35 to about 85 weight %.

Water plays an important role in the MAHTAC monomer synthesis because the monomer is hygroscopic and also because water promotes side reactions. It has been found that the shelf life of the CHPM solutions decreased significantly when moisture was present. Trace water promotes hydrolysis and transesterification reactions in the basic environment and its presence should be avoided.

Preferably, the reaction product from step (a) is reacted with trimethylamine in the presence of the MAHTAC mixture at a temperature of about 25 to about 90 °C. More preferably, the reaction temperature is about 50 to about 85 °C; most preferably, the reaction temperature is about 55 to about 70 °C. Preferably, the reaction time is about 2 to about 48 hours. More preferably, the reaction time is about 3 to about 24 hours; most preferably, the reaction time is about 4 to about 10 hours.

Preferably, the reaction occurs in the presence of a polymerization inhibitor. Preferred polymerization inhibitors are p-methoxyphenol, hydroquinone, and phenothiazine; these materials are available in commercial quantities. The polymerization inhibitors may be used in conventional amounts.

Because the 3-methacryloyloxy-2-hydroxypropyltrimethylammonium chloride monomer acts as a nucleus or seed to influence the particle size of the product, the sizes of the resulting crystals are larger than were found when using the prior art. The particle size is greater than about 40 microns. Preferably, the

particle size is about 40 to 200 microns. Because the particle size is larger, the monomer is readily filtered out of the reaction medium so as to provide a high purity product. "high purity" as used herein means a product with low levels of 2-hydroxypropane-1,3-dimethacrylate (HPDMA);
3-chloropropane-1,2-dimethacrylate (CPDMA);
3-chloro-2-hydroxypropyl methacrylate (CHPM);
glycidylmethacrylate (GMA); and 2,3-dihydroxypropyl methacrylate (DHPMA).

In addition to acting as a catalyst in steps (a) and (b) and as a seed in step (b), the use of MAHTAC improves the overall production economics by eliminating catalyst cost. Preferably, the formed MAHTAC mixture is recycled back to step (a) for use as the catalyst therein. Thus, these monomers are not only the final product but they also act as catalysts and seed to enhance the overall reaction.

The present high purity crystalline water-soluble monomers may be used in the preparation of water-soluble or polymers or copolymers having antistatic, flocculation and water treatment properties.

The present invention is more fully illustrated by the following non-limiting Examples in which unless otherwise noted all parts are parts by weight and all temperatures are expressed in degrees Celsius.

In the Examples of the step (a) preparation, the composition of the CHPM reaction mass was investigated by gas chromatography. An analytical curve which included the compounds: epichlorohydrin (EPI); methacrylic acid (MAA); gllycidylmethacrylate (GMA); 1,3-dichloropropanol (DCP); chloropropane-1,2-diol (CPD); 3-chloro-2-hydroxypropyl methacrylate (CHPM); 1-chloro-3-hydroxypropane2-methacrylate [CHMP-Isomer); p-methoxphenol (MEHQ); 2,3-dihydroxypropyl methacrylate (DHPMA); 3-chloropropane-1,2-dimethacrylate (CPDMA); glyceryl trimethacrylate (GTMA); and 2-hydroxypropanel,3-dimethacrylate (HPDMA) was prepared for quantitative and qualitative determination of the reaction mass. The concentration of the standards ranged from 100 to 900 ppm.

## Example 1

This Example is directed to the preparation of 3-chloro-2-hydroxyproppylmethacrylate (CHPM).

To a 500 mL. 3-necked round bottomed flask, equipped with a thermometer, agitator and reflux condenser, are charged 172.31 grams (2 moles) of methacrylic acid (MAA), 194.40 grams (2.1 moles) of epichlorohydrin (EPI), 0.86 gram (0.5 weight % of MAA) of p-methoxyphenol as polymerization inhibitor and 9.52 grams (0.04 mole) of 3-methacryloyloxy-2-hydroxypropyltrimethylammonium as catalyst. The reaction mixture is heated to 85°C for 7 hours. A slight exotherm is observed in the first hour of the reaction. The yield of the CHPM is 95% based on methacrylic acid and the purity of the CHPM was about 88.27%. Gas chromatographic analysis shows that the CHPM solution has the following composition (weight percent): CHPM 88.27%, EPI 1.27%, MAA 0.67% DCP 3.97%, GMA 0.68%, HPDMA 1.22%, MEHQ 0.24%, CPDMA 0.18%, DHPMA 0.95%, and catalyst 2.55%.

The crude CHPM solution is then extracted with a saturated aqueous $NaHCO_3$ until the disappearance of acid reaction (to remove residual methacrylic acid), then with a 2% sulfuric acid, and after that with water. After vacuum distillation to remove residual epichlorohydrin and water, a light yellow CHPM solution with greater than 97% purity is obtained.

## EXAMPLES 2-4

Examples 2-4 are directed to the preparation of 3-chloro-2-hydroxypropylmethacrylate (CHPM).

The procedure of Example 1 is repeated under the conditions shown in Table 1 below. Each reaction is conducted in the presence of 0.43 gram (0.5 weight % of MAA) of p-methoxyphenol. In order to minimize exothermic reaction, the reaction temperature at the first hour is kept at 65°C and then gradually raised to 85%C. The yield of the CHPM is calculated. The reaction rate constant (K) in each Example is the slope of the plot of -1n ([MAA]/[MA]) vs reaction time. All values are in mole percent. The results are in Table 1 below.

Table 1

| Exp | [EPI]/[MAA] | [MAHTAC]/[MAA] | Temp. °C | Time (Hrs) | K | %Yield |
|---|---|---|---|---|---|---|
| 2 | 1.05 | 0.02 | 75 | 7 | 0.3179 | 97 |
| 3 | 1.05 | 0.03 | 75 | 6 | 0.4581 | 99 |
| 4 | 1.05 | 0.04 | 75 | 5 | 0.5287 | 97 |

Plotting [MAHTAC]/[MAA] against K indicates that K in the formation of CHPM is a linear function of the MAHTAC catalyst concentration.

## EXAMPLE 5

This Example is directed to the preparation of 3-methacryloyloxy-2-hydroxypropyltrimethylammonium chloride.

To a 500 mL. Fisher & Porter pressure bottle equipped with a magnetic stirrer are charged 151 grams (0.746 mole) of unpurified CHPM solution from Example 1, 154.05 grams of acetonitrile, 0.12 gram of p-methoxyphenol, and 49.5 grams (0.837 mole) of trimethylamine. The bottle is sealed and heated in an oil bath at 65 °C for 10 hours. The reaction mixture starts to turn cloudy after 30 minutes of stirring. A white crystalline MAHTAC monomer is obtained with a 98% yield. The average particle size of MAHTAC monomer product is 30 to 40 microns. FIG. 1 is an optical micrograph of the monomer product at a magnification of 130X. Liquid Chromatographic analysis shows that the white crystal is a composition of 94.9% of MAHTAC monomer, 3% of 2-hydroxypropane-1,3-bistrimethylammonium chloride (DIQUAT), and less than 1.9% of 2,3-dihydroxypropyltrimethylammonium chloride (DHPTAC). Other impurities determined by liquid chromatography of ether extracts are MAA 12ppm., GMA 18 ppm., CHPM 350 ppm., DHPMA 87 ppm., and HPDMA 2244 ppm.

## Example 6

This Example illustrates the seeding effect of the MAHTAC monomer in the second step of the MAHTAC monomer preparation.

A procedure similar to that of Example 5 is followed. During the course of quaternization, a small amount of fine MAHTAC crystals in an amount of 5 weight % of CHPM is added into the batch liquor (super saturated solution). It is found that the average particle size of crystals increases to 200 microns and impurity levels dropped by a factor of four. FIG. 2 is an optical micrograph of the monomer product at a magnification of 130X.

## Example 7

This example demonstrates the high purity of MAHTAC made by the process of the present invention. The following two experiments were carried out in an agitated, 7-liter, stainless steel, jacketed, pressure reactor, fitted with temperature and pressure instrumentation.

### Experiment A

To the above reactor are added 400 grams of MAHTAC, 1500 grams of CHPM, 2600 grams of acetonitrile and 500 grams of trimethylamine. The reactor is heated to 50 °C and allowed to react for 19.5 hours. The contents of the reactor are then added to a 12 inch diameter, perforate basket centrifuge containing a filter teflon cloth and separated from the mother liquor (ML) at 1960 Rpm. They are then

washed with 330 grams of acetonitrile at low speed followed by spinning again at 1960 Rpm. The crystals are viewd under the microscope and found to have a mean size of 57.8 microns. The impurity levels for both the mother liquor and the washed crystals are listed in Table II below.

Experiment B

To the above reactor are added 120 grams of glycidyl methacrylate, 1500 grams CHPM, 2600 grams acetonitrile and 500 grams TMA. The reactor is heated to 50° C for 5 hours. Following the procedure in the above experiment the reactor contents are centrifuged and washed. Microscopic examination of the crystals showed that they had a mean particle size of 16.5 microns. The impurity levels are listed in Table II below.

Table II

| | Experiment A (Large Crystal) | | Experiment B (Small Crystal) | |
|---|---|---|---|---|
| Impurity | ML(%) | (ppm) | ML(%) | (ppm) |
| DHPMA | 0.21 | 2.5 | 0.27 | 5.0 |
| MAA | 0.38 | 0.5 | 0.62 | 1.5 |
| CHPM | 1.6 | 28 | 1.2 | 95 |
| GMA | 1.2 | ND | 4.6 | 44 |
| HPDMA | 2.3 | 22 | 1.2 | 50 |
| CPDMA | 4.9 | 35 | 4.8 | 130 |
| GTMA | 0.76 | 12 | 0.78 | 35 |

Table II shows the levels of the impurities found in the crystal mother liquor and in the washed crystal for the two experiments of Example 14. These experiments shown that a significant reduction (up to 73%) in crosslinking impurities can be achieved by increasing the crystal size from 16.5 to 57.8 microns.

**Claims**

1. A mixture of crystalline 3-methacryloyloxy-2-hydroxypropyltrimethylammonium chloride monomer and its isomer, 2-methacryloyloxy-3-hydroxypropyltrimethylammonium chloride monomer, wherein said crystalline monomers have an average particle size greater than 40 microns, said crystals being directly prepared without an intervening recrystallization step.

2. The mixture of claim 1 wherein said particle size is about 40 to 200 microns.

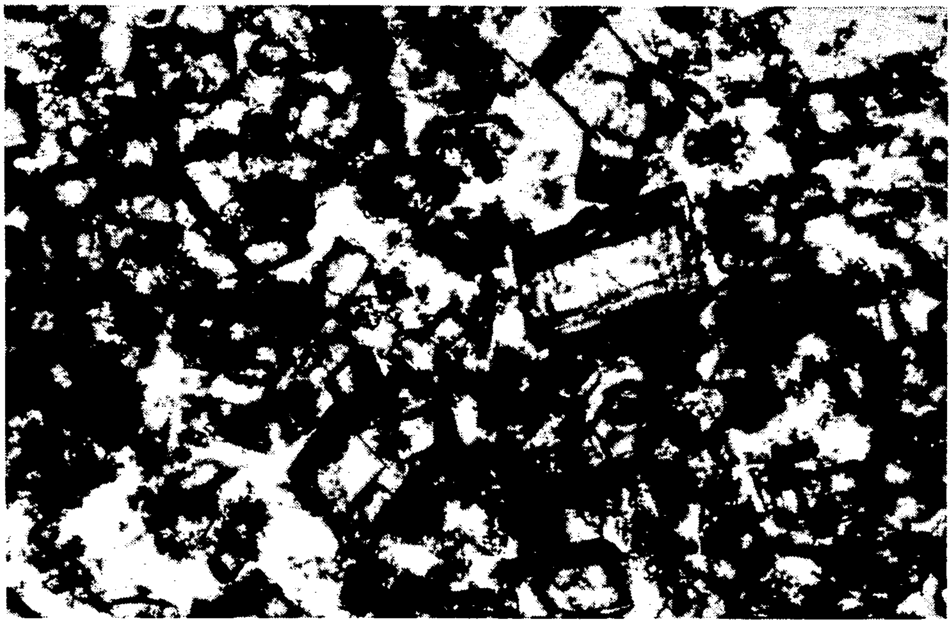

FIG. 1

100μ

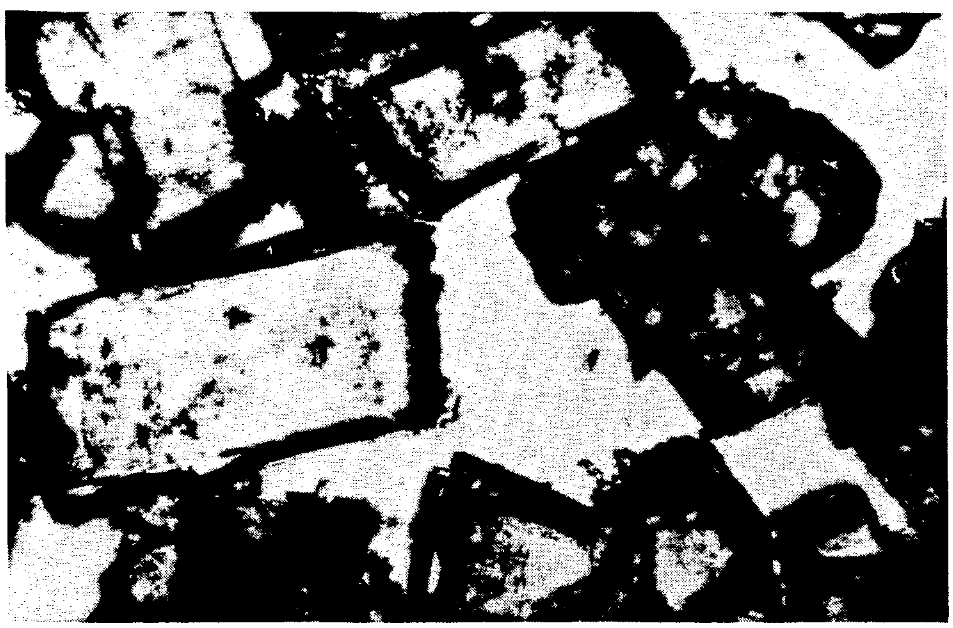

FIG. 2

100μ

European Patent Office

# EUROPEAN SEARCH REPORT

Application Number

EP 89 31 2528

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DE-A-1 593 421 (SHELL)<br>* Example 2, examples 4-6 * | 1,2 | C 07 C 213/02<br>C 07 2 19/08 |
| X | CHEMICAL ABSTRACTS, vol. 102, no. 20, 20th May 1985, page 14, abstract no. 167349g, Columbus, Ohio, US; & PL-A-119 898 (POLITECHNIKA SLASKA) 30-01-1982<br>* Abstract * | 1,2 | |

TECHNICAL FIELDS SEARCHED (Int. Cl.5)

C 07 C 213/00
C 07 C 219/00

The present search report has been drawn up for all claims

| Place of search | Date of completion of the search | Examiner |
|---|---|---|
| THE HAGUE | 22-02-1990 | WELLS A.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)